# EUROPEAN PATENT SPECIFICATION

(11) **EP 4 268 874 B1**
(45) Date of publication and mention of the grant of the patent: **13.08.2025**
(21) Application number: 23168324.4
(22) Date of filing: 17.04.2023
(51) Int. Cl.: A61M 16/12, A61M 11/04, A61M 16/18, A61M 16/20, A61M 16/00, A61M 16/10

(54) **ANESTHETIC VAPORIZER GAS MIXER AND SYSTEM**
NARKOSEMITTELVERDAMPFER-GASMISCHER UND SYSTEM
UN MÉLANGEUR DE GAZ À VAPORISATEUR D'ANESTHÉSIQUE ET SYSTÈME

(30) Priority: 28.04.2022 US 202217661253
(43) Date of publication of application: 01.11.2023
(73) Proprietor: GE Precision Healthcare LLC, Wauwatosa, WI 53226 (US)
(72) Inventor: LACEY, Joseph J., Madison, 53714 (US)
(74) Representative: Kilburn & Strode LLP

(56) References cited:
- DE-A1- 2 657 425
- GB-A- 191 408 838
- US-A1- 2021 008 327

## Description

### FIELD

Embodiments of the subject matter disclosed herein relate to anesthesia systems, and more particularly, to systems for an anesthetic vaporizer gas mixer.

### BACKGROUND

During some medical procedures, such as surgical procedures, a patient may be placed under general anesthesia by administration of an anesthetic agent. In some examples, the anesthetic agent may be a volatile anesthetic agent that is administered to the patient via an anesthetic vaporizer. For example, the anesthetic vaporizer may induce and control vaporization of the volatile anesthetic agent from a liquid form. A carrier gas (e.g., a mixture of oxygen and fresh air) may flow into the vaporizer and blend (e.g., mix and converge) with the anesthetic agent vapors generated by the vaporizer. An amount of carrier gas flowing into the vaporizer may be adjusted by an operator of the vaporizer (e.g., an anesthesiologist) in order to adjust a ratio of carrier gas to anesthetic agents within the vaporizer. The mixed gases may then flow to the patient, where they may be introduced via inhalation, for example. The concentration of the anesthetic agent in the mixed gases may be controlled to ensure sufficient anesthetic agent is provided for patient comfort without compromising patient safety.

In US2021/008327, systems and methods are provided for delivering anesthetic agent to a patient. In one example, an anesthetic vaporizer includes a housing defining a sump, the sump configured to hold a self-contained supply of liquid anesthetic agent, a heating element electrically coupled to an electrical mating component, a gas inlet passage and a gas outlet passage, a manifold fluidically coupled to the gas inlet passage and the gas outlet passage, the manifold coupled to the housing and forming a gas-tight seal with the sump, and a quick disconnect pneumatic system coupled to the gas inlet passage and the gas outlet passage, sealing the gas inlet passage and the gas outlet passage from atmosphere.

The following documents are also disclosing anesthetic mixer, namely DE 26 57 425 A1 and GB 08838 A.

### OVERVIEW

An invention is set out in the claims.

### BRIEF DESCRIPTION

In one aspect, a system for a vaporizer gas mixer may include a one-piece body; a gas inlet and a gas outlet; a first flow path fluidically coupled to the gas inlet and the gas outlet, the first flow path having a plurality of curves; and a second flow path fluidically coupled to the first flow path and a vaporizing chamber, wherein the first flow path and the second flow path merge after the gas inlet passage and before the plurality of curves. In this way, by using in-plane and out-of-plane mixing features, homogeneous gas mixing in a short distance is achieved.

It should be understood that the brief description above is provided to introduce in simplified form a selection of concepts that are further described in the detailed description. It is not meant to identify key or essential features of the claimed subject matter, the scope of which is defined uniquely by the claims that follow the
detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1 schematically shows an example of an anesthesia machine.
FIG. 2 schematically shows an example of an anesthetic vaporizer that may be included in an anesthesia machine.
FIG. 3 shows a perspective view of an example of a gas mixer for an anesthetic vaporizer.
FIG. 4 shows a cross-sectional view of a gas mixer for an anesthetic vaporizer, illustrating an example of a gas flow path.
FIG. 5 shows a cross-sectional view of a gas mixer for an anesthetic vaporizer, illustrating an example of a seat for a valve and flow path access for point sensors.
FIG. 6 shows a cross-sectional view of a gas mixer for an anesthetic vaporize, illustrating an example of the gas flow path.
FIG. 7 schematically shows an example of a first mixing passage for in-plane gas mixing.
FIG. 8 schematically shows a cross-section of an example of a flow path illustrating gas flow rotation around a circular bend.
FIG. 9 schematically shows an example of a second mixing passage for out-of-plane gas mixing.
FIG. 10 is a flow chart illustrating an exemplary method for detecting concentration of a mixed gas in an anesthetic vaporizer.

### DETAILED DESCRIPTION

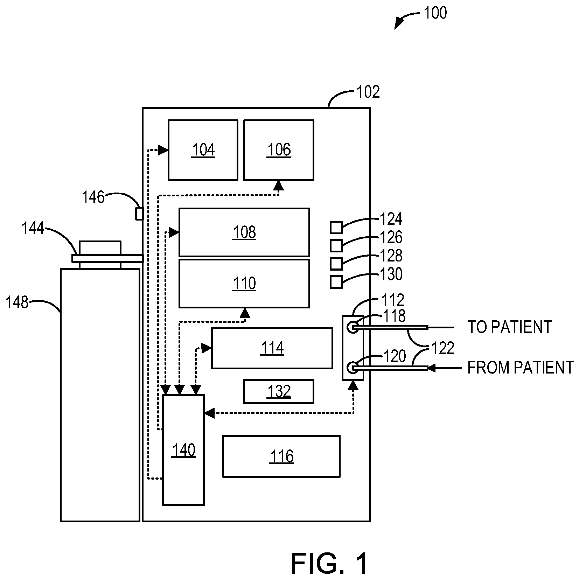

Aspects of the present disclosure will now be described, by way of example, with reference to the FIGS. 1-10, which relate to mixing gas in an anesthetic vaporizer. Anesthetic gases of an anesthetic agent (also herein referred to as an agent or anesthetic gas) may be intentionally generated for delivery to a patient using an anesthetic vaporizer included in an anesthesia machine. Anesthesia vaporizers often mix with medical gas (e.g., purified fresh air, O₂ rich air) anesthetic gases having significantly larger molecular weights (e.g., 6 ~ 8 times heavier than air or O₂). Additionally, anesthetic vaporizers often mix gases having a large dynamic flow range from very low laminar flow to high velocity turbulent flow. Dynamic and verifiable response to user adjustments and precise feedback control may constrain mixing the agent with the medical gas over short mixing lengths. Competing interests in achieving the desired operation include the preference for homogenous mixing, and mixing minimal pressure loss through the gas mixer to prevent error in reading anesthetic agent concentration, limited pressure available in the system, and limited packaging space including between a vaporizing chamber and vaporizer housing (the components above and below the mixture that limits space).

Some fluid (e.g., gas, liquid) mixing methods include corkscrew static mixing. Within a pipe for example, as the mixture of gases flow around a corner, the velocity on the outside radius increases causing the pressure to decrease to balance energy. When the flow reaches a 90 degree turn (e.g., a corner), a pressure difference between the inner radius and the outer radius of the pipe generates rotating flow and increases mixture. Corkscrew static mixing, however, may be less effective when one or more of the gases is very heavy, and in some examples, the heavier gas may centrifuge out of mixture. Another strategy for mixing fluids includes the use of serpentine flow paths (e.g., s-turns or s-curves). Serpentine flow paths include an advantage of attraction of heavier gases to the outside wall of the flow path (e.g., in a pipe). Following an s-turn, the heavier gas will cross through the center line of the flow path to get to the outside wall. Back and forth crossing through the center line mixes the gases of different densities. In some examples, s-turns may increase within layer mixing but not between layer mixing (e.g., stratified in a laminar flow regime). With enough length, heavier gases may mix by diffusion. However, in constrained space (e.g., short mixing length), additional strategies to provide homogenous mixing may be desirable. In some examples, introducing baffles (e.g., obstacles to flow) may increase gas mixing. However, baffles have a disadvantage of reducing pressure in a system.

Thus, according to the examples described herein, a gas mixer for an anesthetic vaporizer system that may be included in an anesthesia machine is provided. Examples of the gas mixer disclosed herein include a non-straight, sinuous three-dimensional mixing path to achieve efficient mixing over a short distance.

Examples disclosed herein may include a one-piece body. The one-piece body may include a gas inlet and a gas outlet. The one-piece body may also include a first flow path fluidically coupled to the gas inlet passage and the gas outlet passage, and a second flow path fluidically coupled to the first flow path. Further, the one-piece body may include a vaporizing chamber. According to other examples, the body may include two or more pieces that are rigidly attached.

The examples described herein may further include the first flow path and the second flow path having gas flows combined after the gas inlet and before a plurality of curves. In one example, the three-dimensional mixing path (or mixing path) uses in-plane mixing, out-of-plane mixing, and/or rotation to enable homogenous mixing over short mixing length. In an example with both in-plane and out-of-plane mixing, it may be advantageous to have a first set of curves in the passage only provide in-plane mixing, and a second set of curves in the passage only provide out-of-plane mixing. Such a configuration takes advantage of limited passage length to provide mixing of different gas density gasses in a more predictable way.

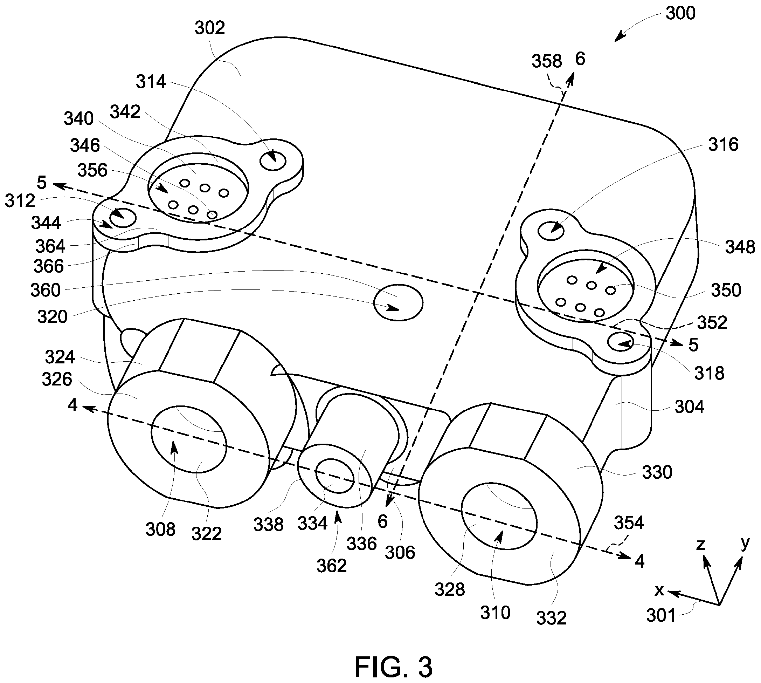

In-plane mixing may be encouraged by the use of a first mixing passage including at least two s-curves in-plane with respect to a gas inlet passage and a gas outlet passage of the system (e.g., X-Y plane in FIG. 3). The first mixing passage includes a circular cross-section for generating rotational flow around the corner or turn to encourage out-of-plane mixing. Out-of-plane mixing is further encouraged by use of a second mixing passage including a curve configured as an out-of-plane turn with respect to the gas inlet passage and the gas outlet passage. The out-of-plane turn, incorporated in another plane (e.g., the Y-Z plane of FIG. 3) perpendicular to the plane of the in-plane mixing, breaks up laminar layers that may form in the Y-Z plane such as in low flow fluids (e.g., low Reynolds number [RE] flows). The plurality of curves generated by the sinuous path of the mixing passages encourages anesthetic agent and medical gas mixture via momentum.

An amount of agent in a mixed gas may be controlled by adjusting a valve positioned between the vaporizing chamber and a valve seat of the gas mixer, the adjustment based on a signal difference measured at a first sensor assembly and a second sensor assembly. The examples described herein further include a method for operating feedback control for adjusting the agent concentration of mixed gas delivery based on sensor signals and generating an indication alerting an operator of the anesthetic vaporizer to detected difference in expected agent concentration.

The examples disclosed herein may provide several advantages. For example, the examples disclosed mix gases over a large dynamic flow range quickly, homogeneously, and with reduced pressure drop over a short mixing length. The thorough mixing with reduced pressure drop enables the use of point concentration sensors for determining agent concentration in the mixed gas. Point concentration sensors may be small, solid state devices, allowing for size reductions in package design. Given particular positioning and packaging constraints in the overall system, in-plane and out-of-plane mixing are provided in short distance while also still meeting packaging constraints and sensing requirements before and after the mixing. Moreover, achieving homogenous mixture over a short mixing length reduces system response lag allowing for more precise agent delivery and automatic feedback control.

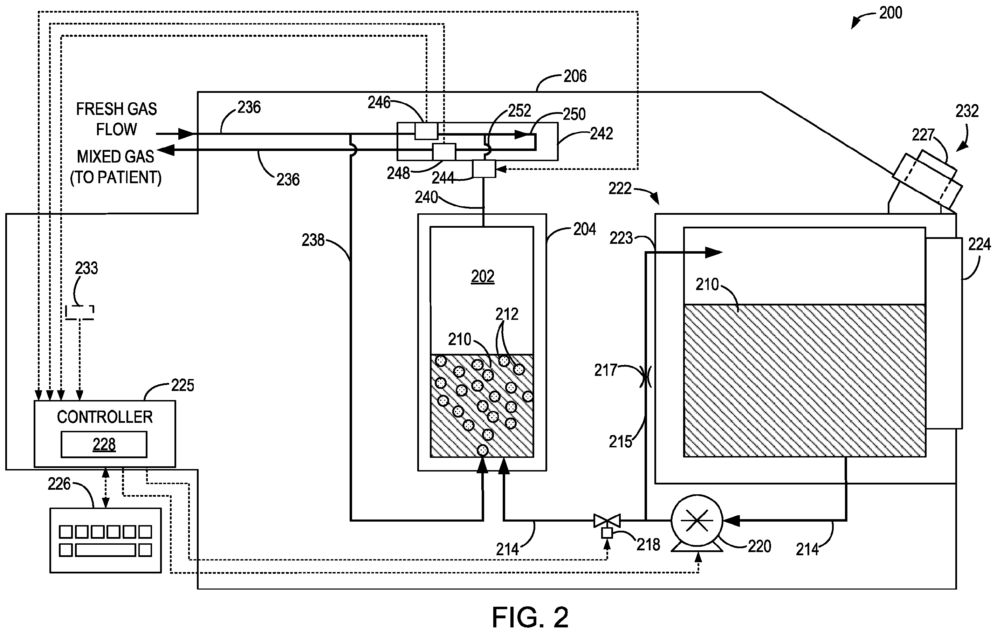

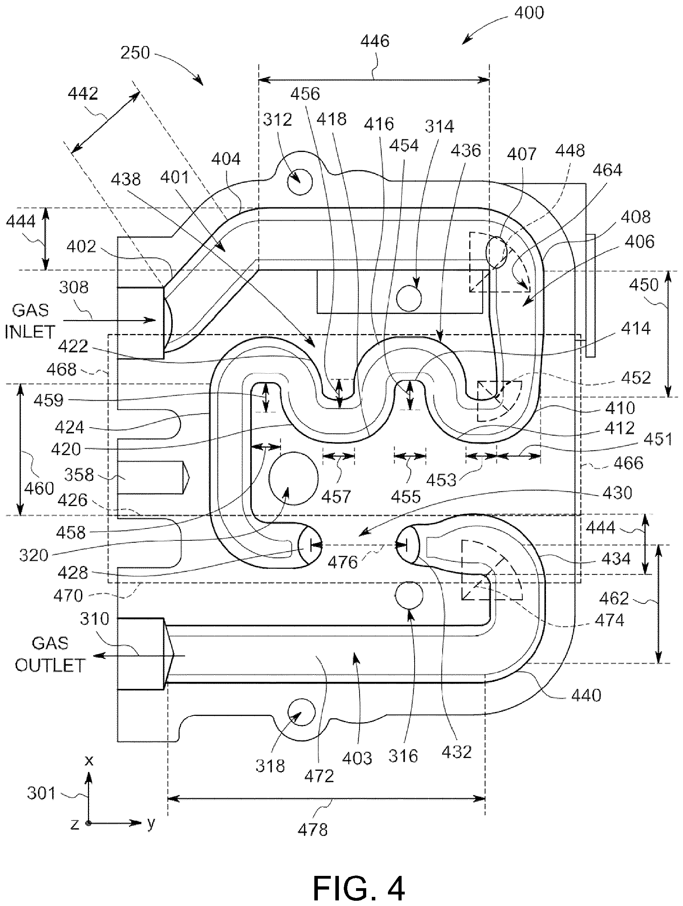

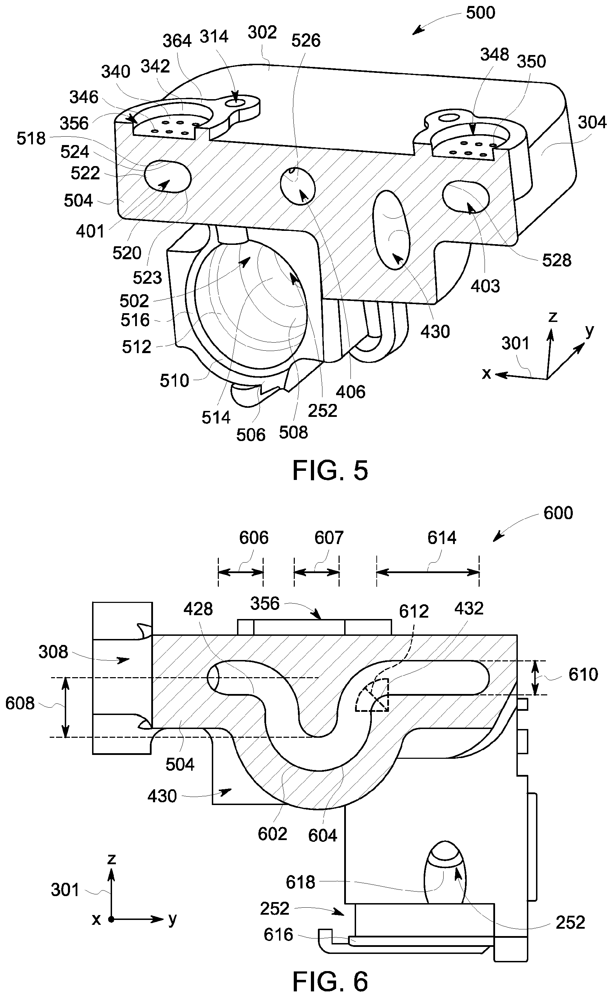

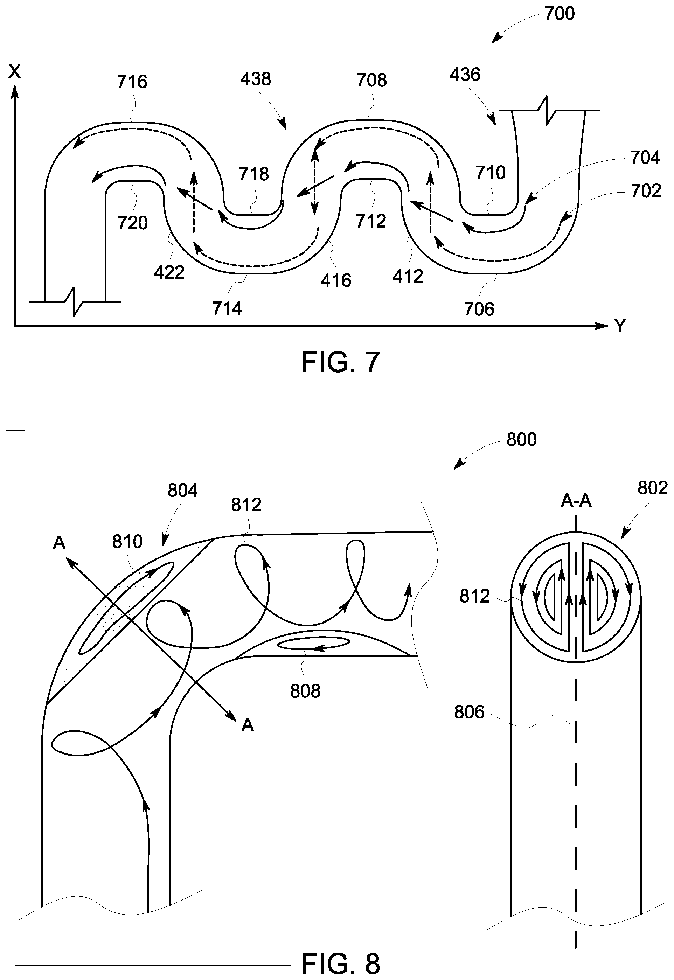

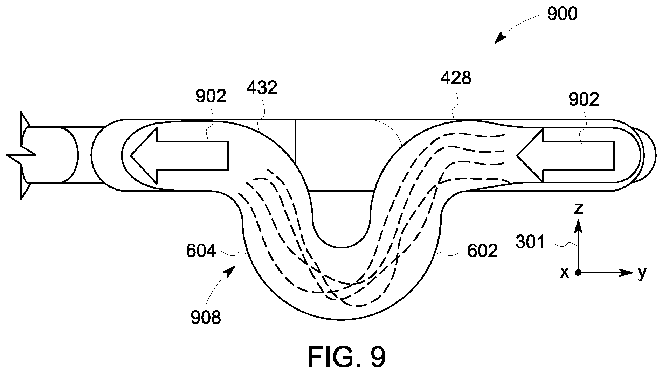

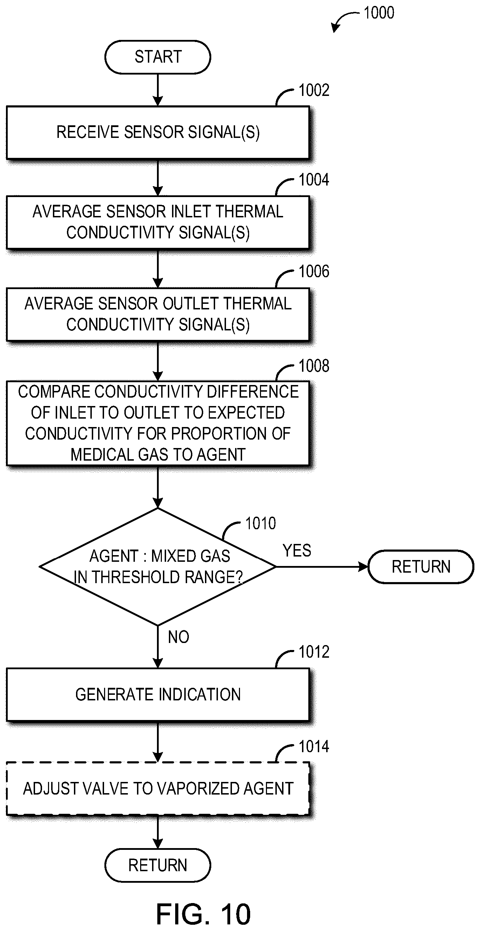

FIG. 1 schematically shows an example of an anesthesia machine. FIG. 2 shows an example of an anesthetic vaporizer that may be included in the anesthesia machine of FIG. 1. In particular, the anesthetic vaporizer includes a gas mixer in electronic communication with a control unit via a first sensor assembly and a second sensor assembly that determine an amount of agent in a mixed gas, and a valve for actuating a flow of agent from a vaporizing chamber to the gas mixer. FIG. 3 shows a perspective view of an exemplary embodiment of a gas mixer, which may be included in the anesthetic vaporizer of FIG. 2. FIG. 4 is a first cross-section illustrating an example of a gas flow path for a gas mixer. FIG. 5 is a second cross-section illustrating an example of a seat for a valve and flow path access for point sensors for a gas mixer. FIG. 6 show a third cross-section illustrating an example of a gas flow path for a gas mixer. FIG. 7 schematically shows an example of a first mixing passage including in-plane s-curves. FIG. 8 schematically shows gas flow rotation around a circular bend, such as described with respect to an aspect of the first mixing passage. FIG. 9 schematically shows an aspect of a second mixing passage including a curve configured as an out-of-plane turn. FIG. 10 shows an example method for controlling an agent concentration of mixed gas in an anesthetic vaporizer based on a signal difference measured at a first sensor assembly and a second sensor assembly. Thus, methods and systems are provided for homogenous mixing of gases over a short mixing length enabling dynamic and precise feedback control.

Turning now to the figures, FIG. 1 schematically shows an example anesthesia machine 100. The anesthesia machine 100 includes a frame (or housing) 102. In some examples, the frame 102 may be supported by casters, where the movement of the casters may be controlled (e.g., stopped) by one or more locks. In some examples, the frame 102 may be formed of a plastic material (e.g., polypropylene). In other examples, the frame 102 may be formed of a different type of material (e.g., metal, such as steel).

The anesthesia machine 100 also includes an anesthesia display device 104, a patient monitoring display device 106, a respiratory gas module 108, one or more patient monitoring modules, such as a patient monitoring module 110, a ventilator 112 (explained in more detail below), an anesthetic vaporizer 114, and an anesthetic agent storage bay 116. The anesthesia machine 100 may further include a main power indicator 124, a system activation switch 126 (which, in one example, permits gas flow when activated), an oxygen flush button 128, and an oxygen control 130. An aspect of the anesthetic vaporizer 114 will be described below with respect to FIGS. 2-9. The anesthetic vaporizer 114 may vaporize the anesthetic agent and combine the vaporized anesthetic agent with one or more medical grade gases (e.g., oxygen, air, nitrous oxide, or combinations thereof), which may then be delivered to a patient.

The anesthesia machine 100 may additionally include an integrated suction, an auxiliary oxygen flow control, and various other components for providing and/or controlling a flow of the one or more medical grade gases to the patient. In the example shown, the anesthesia machine 100 includes one or more pipeline connections 146 to facilitate coupling of the anesthesia machine to pipeline gas sources. Additionally, the anesthesia machine 100 includes a cylinder yoke 144, via which one or more gas-holding cylinders 148 may be coupled to the anesthesia machine. Thus, through the pipeline connection and/or cylinder connections, gas may be provided to the anesthesia machine, where the gas may include (but is not limited to) medical air, oxygen, nitrogen, and nitrous oxide. The gas that enters the anesthesia machine may mix with the vaporized anesthetic agent at the anesthetic vaporizer 114, as described above, before being supplied to a patient via ventilator 112. The anesthesia machine may also include a serial port, a collection bottle connection, and a cylinder wrench storage area. Further, in some examples, the anesthesia machine may include an anesthesia gas scavenging system 132.

The ventilator 112 may include an expiratory check valve at an expiratory port 120, an expiratory flow sensor at the expiratory port 120, an inspiratory check valve at an inspiratory port 118, an inspiratory flow sensor at the inspiratory port 118, an absorber canister, a manual bag port, a ventilator release, an adjustable pressurelimiting valve, a bag/vent switch, and a bellows assembly. When a patient breathing circuit is coupled to the ventilator 112, breathing gases (e.g., air, oxygen, and/or nitrous oxide mixed with vaporized anesthetic agent) exit the anesthesia machine from the inspiratory port 118 and travel to the patient. Expiratory gases from the patient re-enter the anesthesia machine via expiratory port 120, where carbon dioxide may be removed from the expiratory gases via absorber canister.

During operation of the anesthetic vaporizer 114, an operator (e.g., an anesthesiologist) may adjust an amount of vaporized anesthetic agent that is supplied to the patient by adjusting a flow rate of gases from the gas source(s) (e.g., the pipeline gas supply) to the vaporizer. The flow rate of the gases from the gas source to the vaporizer may be adjusted by the operator via one or more flow adjustment devices. For example, the flow adjustment devices may include analog and/or digital adjustment dials and/or other user input devices configured to actuate one or more flow control valves of the anesthesia machine 100. In some examples, a first flow control valve may be positioned between the gas source(s) and the anesthetic vaporizer 114 and may be actuatable via flow adjustment devices to a fully open position, a fully closed position, and a plurality of positions between the fully open position and the fully closed position.

The anesthesia machine 100 may additionally include one or more valves configured to bypass gases from the gas source(s) around the anesthetic vaporizer 114. The valves may enable a first portion of gases to flow directly from the gas source to the inspiratory port 118 and a second portion of gases to flow from the gas source through the anesthetic vaporizer 114 to mix with the vaporized anesthetic agents prior to flowing to the inspiratory port 118. By adjusting a ratio of the first portion of gases relative to the second portion of gases, the operator may control a concentration of vaporized anesthetic agent administered to the patient via inspiratory port 118.

Further, the adjustments described above may be facilitated at least in part based on output from the respiratory gas module 108. The respiratory gas module 108 may be configured to measure various parameters of the gases exiting the vaporizer and/or being provided to the patient. For example, the respiratory gas module 108 may measure the concentrations of carbon dioxide, nitrous oxide, and the anesthetic agent provided to the patient. Further, the respiratory gas module 108 may measure respiration rate, minimum alveolar concentration, patient oxygen, and/or other parameters. The output from the respiratory gas module 108 may be displayed via a graphical user interface on a display device (e.g., the anesthesia display device 104 and/or the patient monitoring display device 106) and/or used by a controller to provide closed-loop feedback control of the amount of anesthesia provided to the patient.

The ventilator 112 may optionally be coupled to a breathing circuit (not shown) including a plurality of tubes (e.g., gas passages) 122. The breathing circuit may be coupled between an airway of a patient (e.g., via a breathing mask positioned to enclose the mouth and/or nose of the patient or a tracheal intubation tube) and the inspiratory port 118. Gases (e.g., the one or more medical gases, or a mixture of the one or more medical gases and vaporized anesthetic agent from the anesthetic vaporizer 114) may flow from the inspiratory port 118, through the breathing circuit, and into the airway of the patient, where the gases are absorbed by the lungs of the patient. By adjusting the concentration of vaporized anesthetic agent in the gases as described above, the operator may adjust a degree to which the patient is anesthetized.

During conditions in which the breathing circuit is coupled to the airway, the anesthetic agent and/or fresh gas (without the anesthetic agent) may flow into the airway of the patient (e.g., through inhalation) via inspiratory port 118 and the inspiratory check valve. As an example, the inspiratory check valve may open automatically (e.g., without input or adjustment by the operator) in response to inhalation by the patient and may close automatically in response to exhalation by the patient. Similarly, the expiratory check valve may open automatically in response to exhalation by the patient and may close automatically in response to inhalation by the patient.

In some examples, the operator may additionally or alternatively control one or more operating parameters of the anesthesia machine 100 via an electronic controller 140 of the anesthesia machine 100. The controller 140 includes a processor operatively connected to a memory. The memory may be a non-transitory computerreadable medium and may be configured to store executable instructions, e.g., computer executable code, to be processed by the processor in order to execute one or more routines, such as those described herein. The memory may also be configured to store data received by the processor. The controller 140 may be communicatively coupled (e.g., via wired or wireless connections) to one or more external or remote computing devices, such as a hospital computing system, and may be configured to send and receive various information, such as electronic medical record information, procedure information, and so forth. The controller 140 may also be electronically coupled to various other components of the anesthesia machine 100, such as the anesthetic vaporizer 114, the ventilator 112, the respiratory gas module 108, the anesthesia display device 104, and the patient monitoring display device 106.

The controller 140 receives signals from the various sensors of the anesthesia machine 100 and employs the various actuators of the anesthesia machine 100 to adjust operation of the anesthesia machine 100 based on the received signals and instructions stored on the memory of the controller. For example, the flow of gases to the inspiratory port 118 may be controlled via an input device (e.g., keyboard, touchscreen, etc.) coupled to the electronic controller of the anesthesia machine 100. The controller 140 may display operating parameters of the anesthesia machine 100 via anesthesia display device 104 and/or the patient monitoring display device 106. The controller may receive signals (e.g., electrical signals) via input device and may adjust operating parameters of the anesthesia machine 100 in response (e.g., responsive) to the received signals.

As one example, the operator may input a desired concentration of the anesthetic agent to be delivered to the patient. A corresponding valve position of one or more valves of the anesthesia machine (e.g., a position of one or more bypass valves, as described above) may be empirically determined and stored in a predetermined lookup table or function in a memory of the controller. For example, the controller may receive the desired concentration of the anesthetic agent via an input device and may determine an amount of opening of the one or more valves corresponding to the desired concentration of the anesthetic agent based on the lookup table, with the input being the concentration of the anesthetic agent and the output being the valve position of the one or more valves. The controller may transmit an electrical signal to an actuator of the one or more valves in order to adjust each of the one or more valves to the corresponding output valve position. In some examples, the controller may compare the desired flow rate of gases to a measured flow rate of gases, such as measured by the inspiratory flow sensor, for example.

The controller 140 is shown in FIG. 1 for illustrative purposes, and it is to be understood that controller 140 may be located in various locations within, around, and/or remote from the anesthesia machine 100. As an example, the controller 140 may include multiple devices/modules that may be distributed throughout the anesthesia machine 100. As such, the controller 140 may include a plurality of controllers at various locations within the anesthesia machine 100. As another example, additionally or alternatively, the controller 140 may include one or more devices/modules that are external to the anesthesia machine 100, located proximate to (e.g., in a same room) or remote (e.g., at a remote server) from the anesthesia machine 100. In each example, the multiple devices/modules may be communicatively coupled through wired and/or wireless connections.

Anesthetic vaporizers, such as the anesthetic vaporizer 114 shown in FIG. 1, may employ various methods to vaporize a liquid anesthetic agent. For example, the anesthetic vaporizer 114 may use a flow-over method (in which a carrier gas flows over a top surface of a volatile liquid anesthetic agent), a bubble-through method (in which the carrier gas is bubbled up through the liquid anesthetic agent), or a gas/vapor blender (in which heat is used to vaporize the liquid anesthetic agent, and the vapors are injected into a fresh gas flow). Regardless of the vaporization method, in some examples, the anesthetic vaporizer 114 may include a sump for storing the liquid anesthetic agent before it is delivered to a vaporizing chamber.

FIG. 2 shows an aspect of an anesthetic vaporizer 200 including a vaporizer gas mixer or gas mixer 242, which may be included in an anesthesia machine (e.g., the anesthesia machine 100 shown in FIG. 1). As one example, the anesthetic vaporizer 200 may be the anesthetic vaporizer 114 of FIG. 1. In the example, the anesthetic vaporizer 200 is a bubble-through anesthetic vaporizer, including an anesthetic source or vaporizing chamber 202 defined by a housing 204. However, in other examples, the anesthetic vaporizer 200 may be another type of anesthetic vaporizer (e.g., flow-over, injector-based, wick-based, etc.) for use with a volatile liquid anesthetic agent, and the bubble-through architecture is shown for illustrative purposes.

A lower portion of the vaporizing chamber 202 is shown holding a liquid anesthetic agent 210 that is supplied from a sump 222 via a conduit 214 and a pump 220. The liquid anesthetic agent 210 may be isoflurane, sevoflurane, or another liquid anesthetic agent of similar volatility, for example, that is stored in the sump 222. The pump 220 may be a positive displacement pump, such as a reciprocating positive displacement pump, for example. The pump 220 may be selectively operated to deliver the liquid anesthetic agent 210 from the sump 222 to the vaporizing chamber 202 in response to a command signal from a controller 225, as will be further described below. The controller 225 may be an electronic controller including a processor operatively connected to a memory 228, which may be a non-transitory memory (e.g., read-only memory) that stores instructions executable by the processor. The controller 225 may be included in (e.g., a part of) or communicatively coupled to the controller 140 shown in FIG. 1, for example.

The sump 222 is defined by a housing 223. The housing 223 and the housing 204 may be integrated with or positioned with an external housing 206 of the anesthetic vaporizer 200. For example, the pump 220, the conduit 214, etc. may be internal components within the external housing 206. The sump 222 may be refilled via a filler apparatus 232 positioned on an exterior of the housing 223 and the housing 206. The filler apparatus 232 includes a filler port 227. In some examples, the filler apparatus 232 may further include a fill cap (not shown in FIG. 2) configured to cover the filler port 227 when a refilling event is not occurring. For example, an operator of the anesthetic vaporizer 200 may remove the fill cap to refill the sump 222 with additional liquid anesthetic agent 210 (e.g., from a refill bottle) via the filler port 227 and then replace the fill cap to seal the sump 222. The fill cap may be a screw cap, for example. Thus, in some examples, the sump 222 may be a sealed system when the fill cap is in place. In some examples, a sight glass 224 may enable the operator to evaluate a fill status of the sump 222.

The conduit 214 may further include a shut-off valve 218 coupled between the pump 220 and the vaporizing chamber 202. For example, the shut-off valve 218 may be an on-off valve, wherein the shut-off valve 218 is actuated to an open (e.g., fully open) position that allows the liquid anesthetic agent 210 to flow between and the pump 220 and the vaporizing chamber 202 or a closed (e.g., fully closed) position that prevents (e.g., blocks) the flow of the liquid anesthetic agent 210 between the pump 220 and the vaporizing chamber 202. The shut-off valve 218 may be actuated between the open and closed positions in response to a command signal from the controller 225, for example. A liquid return line 215 may be coupled to the conduit 214 between the shut-off valve 218 and the pump 220 to reduce a pressure build-up between the shut-off valve 218 and the pump 220, such as when the shut-off valve 218 is closed. For example, an excess amount of the liquid anesthetic agent 210 provided by the pump 220 may be returned to the sump 222 via the liquid return line 215. Further, the liquid return line 215 may include a restriction 217, such as an orifice, to control flow through the liquid return line 215 such that the liquid anesthetic agent 210 preferentially flows through the shut-off valve 218 instead of the restriction 217 when the shut-off valve 218 is open.

The controller 225 may selectively activate the pump 220 to provide the liquid anesthetic agent 210 from the sump 222 to the vaporizing chamber 202. In one example, the controller 225 may adjust operation of the pump 220 responsive to a measurement received from a level sensor coupled to the vaporizing chamber 202. As one example, the controller 225 may be configured to maintain the level of liquid anesthetic agent at a target level or within a target range in order to prevent both underfilling and overfilling of the vaporizing chamber 202.

In some examples, the pump 220 may include a positive displacement stepper motor, where each positive displacement step of the pump is equivalent to a specified volume of the liquid anesthetic agent 210. In this manner, the pump may be used to precisely fill the vaporizing chamber 202 and prevent overfilling by recording the number of pump steps delivered. This approach may also be used to record a volume of anesthetic agent delivered to the vaporizing chamber 202, which may be used for vaporizer run-time/maintenance analysis (e.g., service metrics), liquid leak detection, precise determination of an amount of liquid anesthetic remaining and available for delivery, vaporization efficiency calculations, etc.

An upper portion of the vaporizing chamber 202 (e.g., above a surface of the liquid anesthetic agent 210) holds vapor, which may be a mixture of vaporized anesthetic agent and a carrier gas from a fresh gas flow (also herein referred to as a medical gas flow). The fresh gas flow, and thus the carrier gas, may include one or more medical grade gases, such as oxygen, air, nitrous oxide, and combinations thereof. The fresh gas flow may be provided via one or more gas pipelines (e.g., via the pipeline connections 146 shown in FIG. 1) and/or one or more gas-holding cylinders (e.g., the gas-holding cylinder 148 of FIG. 1). As shown in FIG. 2, the fresh gas flow may enter anesthetic vaporizer 200 via a first gas passage 236.

In the example, a second gas passage 238 branches off from first gas passage to provide carrier gas to the vaporizing chamber 202. The first gas passage enters the gas mixer 242 to provide bypass gas to the gas mixer. In the example, the first gas passage 236 from the point of gas mixer entry to gas mixer exit may also be referred to as first flow path 250. As used herein, "carrier gas" refers to a portion of the fresh gas flow that flows to the vaporizing chamber 202, whereas "bypass gas" refers to a remaining portion of the fresh gas flow that does not flow through the vaporizing chamber 202, as will be elaborated below. For example, the second gas passage 238 may pass through an opening in the housing 204, which may include a gas-tight seal, to flow the carrier gas through a bottom of the vaporizing chamber 202. However, in other examples, the anesthetic vaporizer 200 may not include the second gas passage 238, and the carrier gas may not be delivered to the vaporizing chamber 202. For example, the carrier gas may not be delivered to the vaporizing chamber 202 when the liquid anesthetic agent 210 has a relatively low boiling point (e.g., at or around room temperature), such as when the liquid anesthetic agent 210 is desflurane or another liquid anesthetic agent of similar volatility. Additionally or alternatively, the second gas passage 238 may not be included in examples where a different type of anesthetic vaporizer architecture is used (e.g., a flow over type or a gas/vapor blender). Thus, the anesthetic vaporizer shown in FIG. 2 is provided by way of example.

The carrier gas delivered to the vaporizing chamber 202 via the second gas passage 238 flows through the liquid anesthetic agent 210 to form a plurality of gas bubbles 212. The plurality of gas bubbles 212 pass through the liquid anesthetic agent 210, becoming saturated with vaporized anesthetic agent, as they rise to the surface of the liquid. In some examples, a heating element may be coupled to or within the vaporizing chamber 202 to increase a temperature of the liquid anesthetic agent 210 and provide energy for vaporization (e.g., latent heat of vaporization).

Vapor, such as the carrier gas that is saturated with vaporized anesthetic agent, may flow out of the vaporizing chamber 202 via a third gas passage 240 (e.g., a vapor delivery passage). For example, the third gas passage 240 may pass through an opening at or near a top of the housing 204. The third gas passage may couple to the gas mixer via valve 244 (e.g., proportional valve) and form a junction with first flow path 250 to fluidically couple the upper portion of the vaporizing chamber 202 with the first gas passage 236 including the first flow path 250. In the example, the third gas passage 240 from valve 244 to the junction with first flow path 250 may be referred to as second flow path 252 (e.g., within the gas mixer). Upstream of the junction with the second flow path 252 and downstream of the junction with the second gas passage 238, the first gas passage 236 carries the bypass gas portion of the fresh gas flow. The bypass gas does not pass through the vaporizing chamber 202. The bypass gas, containing no vaporized anesthetic agent, and the anesthetic gas from the vaporizing chamber 202, containing the carrier gas saturated with the vaporized anesthetic agent, combine within gas mixer 242 at and downstream of the junction between the first flow path 250 and the second flow path 252. The combined flow e.g., mixed gas, may then be delivered to the patient via an inspiratory limb of a breathing circuit (e.g., via the inspiratory port 118 described with respect to FIG. 1).

In some examples, the gas mixer may include a first sensor assembly 246 coupled to first flow path 250 upstream of the junction with second flow path 252. The gas mixer may include a second sensor assembly 248 coupled to first flow path 250 downstream of the junction with second flow path 252. The first sensor assembly may include at least a first sensor and the second sensor assembly may include at least a second sensor, the first and second sensors configured to measure a concentration of the anesthetic agent in the mixed gas. As one example, the first and second sensor assemblies may include point sensors for detecting thermal conductivity of a gas mixture. A concentration of agent in the gas mixture may be determined based on a difference in thermal conductivity measured at the first sensor assembly compared to the second sensor assembly. As another example, the first and second sensor assemblies may include one or more optical sensors that transmits light of a suitable wavelength (e.g., infrared) through the mixed gas and determines a concentration of the anesthetic agent based on an absorption of the light by the mixed gas. In other examples, the first and second sensor assemblies may include one or more carbon dioxide or oxygen sensors that measure the concentration of the anesthetic agent based on a displacement of the carbon dioxide or oxygen relative to a supplied concentration of carbon dioxide or oxygen in the fresh gas flow. The first sensor assembly 246 and the second sensor assembly 248 may output signals to the controller 225 indicative of the measured concentration of the anesthetic agent (e.g., the concentration of the anesthetic agent vapor) in the mixed gas.

In addition to receiving signals output by the first sensor assembly 246 and the second sensor assembly 248, the controller 225 may receive additional signals, including signals from one or more additional sensors 233 coupled in various locations throughout the anesthetic vaporizer 200. The one or more additional sensors 233 may comprise pressure, temperature, and volatile organic compound (VOC) sensors.

The controller 225 receives the signals from the various sensors of FIG. 2, processes the input data, and employs the various actuators of FIG. 2 to adjust operation of anesthetic vaporizer 200 based on the received signals and instructions stored on a memory of the controller. For example, based on signals received from the first sensor assembly 246 and the second sensor assembly 248, valve 244 may be adjusted to control the flow of vaporized agent into the gas mixer. Additionally, the controller 225 may output an indication to the operator via a human-machine interface (HMI) 226 that is operationally connected to the controller (e.g., via wired or wireless communication) responsive to detecting an agent concentration outside of a threshold range. Further, data may be input to the controller 225 by the operator of anesthetic vaporizer 200 via the HMI 226. Thus, the HMI 226 may include both a user input device and an output device. The user input device may include one or more of a mouse, a keyboard, a voice input device, a touch input device for receiving a gesture from the operator, a motion input device for detecting non-touch gestures and other motions by the operator, and other comparable input devices, as well as associated processing elements capable of receiving user input from the operator. The output device may include one or more of a display (e.g., the anesthesia display device 104 and/or the patient monitoring display device 106 of FIG. 1) for providing visual alerts or text-based messages and a speaker for providing audible alerts or messages.

Lag time between adjusting an amount of agent and reliable detection of the adjustment may be decreased by homogenously mixing gases over a shorter mixing length. In one example, within gas mixer 242, the first flow path 250 may be shaped into a plurality of curves, the curves downstream from the junction with the second flow path 252. The plurality of curves shaping the flow path encourages vaporized agent and bypass gas mixture via momentum. In this way, homogeneous mixture may be achieved over very short mixing length and with reduced pressure drop, enabling close proximity sensor assemblies for rapid detection of agent concentration adjustments. Additionally, reliable gas mixing over a short distance may enable more compact vaporizer design.

FIG. 3 shows a perspective view of an example of a gas mixer 300 for an anesthetic vaporizer such as the anesthetic vaporizer 200 illustrated in FIG. 2. Gas mixer 300 may be the same or similar to gas mixer 242 of FIG. 2, which may be included in an anesthesia machine (e.g., the anesthesia machine 100 shown in FIG. 1). Gas mixer 300 will be described herein with reference to the systems and components depicted in FIGS. 1 and 2, though it should be understood that the gas mixer may be applied to other systems without departing from the scope of this disclosure. Components of gas mixer 300 that are identical to components of gas mixer 242 in anesthetic vaporizer 200 are numbered the same and will not be introduced. An axis system 301 is given in FIG. 3 and the figures following. The x-axis may be referred to as a lateral axis, the z-axis may be referred to as a vertical axis, and the y-axis may be referred to as a longitudinal axis.

Gas mixer 300 may include a one-piece formed body without seams, without adhesives, and without connectors. In one example, the body be formed of a plastic material. The body may also be formed from a different type of material such as metal. In one example, one-piece construction achieves homogenous mixture in a compact design, which may be advantageous for meeting packaging constraints in the overall system. Additionally, one-piece construction may be advantageous by enabling manufacturing methods such as casting or 3D printing of the gas mixer.

The body of gas mixer 300 may be substantially rectangular, meaning that it would be understood to be rectangular by a person skilled in the art without being perfectly rectangular. The body of the gas mixer 300 may further include a length greater than the width and a height approximately half the width. An upper surface 302 is shown may be parallel to a matching lower surface (not shown). A first side surface 304 is shown may be parallel to a second side surface (not shown). A front surface 306 is shown may be parallel to a back surface (not shown). Gas mixer 300 may be substantially asymmetrical along a lateral center line and a longitudinal center line.

A gas inlet 308 and a gas outlet 310 may be formed in the front surface 306. A flow path fluidically couples gas inlet 308 and gas outlet 310 (shown in FIG. 4). Gas inlet 308 is a cavity with an opening formed by inner cylindrical surface 322, outer cylindrical surface 324, and annular surface 326. Gas outlet 310 is a cavity with an opening formed by inner cylindrical surface 328, outer cylindrical surface 330, and annular surface 332. Included in a system (e.g., vaporizer 114 in FIG. 1, vaporizer 200 in FIG. 2), gas inlet 308 may couple to a source of fresh air or blended medical gas (e.g., to a mixer or gas blender via first gas passage 236 in FIG. 2) to provide a flow of bypass gas for mixing with vaporized agent. Gas outlet 310 may couple to a breathing circuit (shown as plurality of tubes 122 in FIG. 1) to provide a flow of mixed gas to a patient. Mounting point 362 for the gas mixer may also be formed into the front surface 306. Mounting point 362 may be a cylinder with a shallow recess formed by an inner cylindrical surface 334, outer cylindrical surface 336, and annular surface 338. A through-hole 320 may be provided for mounting. The through-hole 320 may be formed by an inner cylindrical surface 360, an opening on the upper surface 302 and an opening on the lower surface (not shown).

An inlet sensor assembly mount 356 and outlet sensor assembly mount 348 may be formed in the upper surface. As one example, in a vaporizer system, an inlet sensor assembly (e.g., first sensor assembly 246) and an outlet sensor assembly (e.g., second sensor assembly 248) including one or more point sensors may be integrated with gas mixer 300 via inlet sensor assembly mount 356 and outlet sensor assembly mount 348, respectively. Inlet sensor assembly mount 356 may be formed by lip 344 with an inner cylinder surface 342, a lip top surface 364, and a lip side surface 366. Lip 344 may encircle circular depression 340 and pair of through-holes 312, 314. The pair of through-holes 312, 314 may be used for mounting the inlet sensor assembly to the gas mixer in vaporizer system. A plurality through-holes or inlet sensor access through-holes 346 may be provided in circular depression 340. The inlet sensor access through-holes 346 provide routes for sensors (e.g., point sensors) for sampling gas within the flow path upstream from the junction where vaporized agent combines in the flow path. Outlet sensor assembly mount 348 may be formed similarly as inlet sensor assembly mount 356 including a plurality of through-holes or outlet sensor access through-holes 350 and pair of through-holes 316, 318 for mounting. In the example, six outlet sensor access points and six inlet sensor access points are shown. Fewer or more sensor access points may be included in other examples.

In one example, gas mixer 300 may include a non-straight, three-dimensional flow path for mixing medical gas with a vaporized agent. In one example, the gas mixer system may include a plurality of s-curves including at least two s-curves in-plane and at least one s-curve out-of-plane. A first plane, herein referred to as plane 354, indicates a vertical plane cross-section of gas mixer bisecting the body through the gas inlet 308 and the gas outlet 310. The cross-section is shown in FIG. 4 to illustrate interior passages including two in-plane s-curves (e.g., with respect to the first plane). Plane 352 indicates a lateral plane cross-section of the gas mixer through the inlet sensor assembly and the outlet sensor assembly. The cross-section is shown in FIG. 5 to illustrate an inlet of a second flow path (252 in FIG. 2) and interior passages. Plane 358 indicates a longitudinal plane cross-section through the right third of the gas mixer. The cross-section is shown in FIG. 6 to illustrate an out-of-plane turn in a second mixing passage.

FIG. 4 shows a cross-section view of an example of a flow path for gas mixer 400. Gas mixer 400 may be the same or similar to gas mixer 242 of FIG. 2 and gas mixer 300 of FIG. 3. Components of gas mixer 400 of FIG. 4 that are identical to components of gas mixer 242 and 300 are numbered the same and will not be reintroduced. For example, gas mixer 400 includes first flow path 250, gas inlet 308, gas outlet 310, through-holes 312, 314, 316, 318, 320, and mounting point 362. Further, some components of gas mixer 300 may not be shown, although it may be understood that they may also be included in gas mixer 400.

The cross-section view of gas mixer 400 is shown looking into the z axis to illustrate an example of first flow path 250. First flow path 250 is fluidically coupled to the gas inlet 308 and the gas outlet 310. The first flow path 250 includes a smooth internal wall 472 (e.g., without protrusions or baffles). As shown in FIG. 4, the gas mixer 400 may include in-plane passages (e.g., incorporated in the xy plane) including gas inlet passage 401, gas outlet passage 403, and first mixing passage 406 comprising two s-curves. Gas inlet passage 401 may be the region of first flow path 250 from gas inlet 308 until junction 407 with the second flow path (252 in FIG. 2). First mixing passage 406 may be a region of first flow path 250 from junction 407 until curve 428 takes a substantially right angled turn out-of-plane into a second mixing passage 430 (e.g., detailed in FIG. 6 and FIG. 9). Junction 407 indicates where gas flows from the second flow path may enter to mix with gas flows entering at gas inlet 308 (e.g., bypass gas). Gas flows from the second flow path is shown with arrow 464. Gas outlet passage 403 may be the region of first flow path 250 from curve 440 until gas outlet 310. In one example, the interior passages of the gas mixer, including first flow path 250, gas inlet passage 401, first mixing passage 406, second mixing passage 430, gas outlet passage 403, and the second flow path may be a sinuous chamber formed into the one-piece body. In one example, the first mixing passage 406 may be formed within a first region 468 and the second mixing passage 430 is formed within a second region 470. The first region 468 and second region 470 may be a central region 466 of the one-piece plastic body.

Following gas inlet 308, the first flow path 250 may enter gas inlet passage 401, turning upwards approximately 45 degrees at curve 402. After length 442, gas inlet passage 401 may turn back approximately 45 degrees at curve 404. After length 446 which may be approximately twice as long as length 442, gas inlet passage 401 takes a 90 degree turn at curve 408.

Following junction 407, first flow path 250 enters first mixing passage 406. In one example, the first mixing passage includes first s-curve 436 and second s-curve 438 interposed by first perpendicular length 450 and second perpendicular length 460 (perpendicular lengths so-called with respect to gas inlet passage 401). First perpendicular length 450 and second perpendicular length 460 may be similar. Lengths of passage between substantially 90 degree bends in the first s-curve 436 and second s-curve 438 may be similar. Following first perpendicular length 450, at curve 410, first mixing passage 406 may take a substantially 90 degree turn into first s-curve 436. After length 453, first mixing passage 406 may take another 90 turn at curve 412. After length 454, first mixing passage 406 may take another 90 turn at curve 414. After another length 455, first mixing passage 406 may take a 90 turn at curve 416. After another length 456, at curve 418, first mixing passage 406 may take a 90 degree turn into second s-curve 438. After length 457, first mixing passage 406 may take a 90 degree turn at curve 420. After length 458, first mixing passage 406 may take a 90 degree turn at curve 422. After another length 459, first mixing passage 406 may take another 90 turn at curve 424 to finish the second s-curve 438. After second perpendicular length 460, first mixing passage 406 may take a 90 degree turn at curve 426.

At curve 428, the flow path may make a 90 degree turn out-of-plane turn or curve into second mixing passage 430. Second mixing passage 430 may form a U-shaped bend incorporated in the yz plane between curve 428 and curve 432. In the xyplane, the length 476 separates curve 428 and curve 432. Second mixing passage 430 is described in more detail in FIG. 6 and FIG. 8. After curve 432, second mixing passage 430 may take an in-plane 90 degree turn at curve 434. After length 462, second mixing passage 430 passage may take a 90 degree turn at curve 440 to the gas outlet 310.

The first flow path 250 between gas inlet passage 401 and junction 407 may have first diameter 444. The radius of curvature 448 at curve 408 is similar to first diameter 444. Between curve 408 and curve 410 the diameter of first flow path 250 may narrow to second diameter 451. The radius of curvature 452 at curve 410 may be similar to second diameter 451. Following curve 432, the diameter of the gas outlet passage 403 may widen to first diameter 444. The radius of curvature 474 at curve 434 may be similar to first diameter 444.

In an example, upstream from junction 407, e.g., in length 446 of gas inlet passage 401 between curve 404 and curve 408, one or more point sensors inserted through sensor access points in body of the gas mixer (e.g. inlet sensor access through-holes 346 in FIG. 3) may sample gas in first flow path 250. Sampling upstream from junction 407 may provide a measurement of gas content before addition of a vaporized agent via the second flow path. Downstream from second mixing passage 430, e.g., in length 478 of gas outlet passage 403 between curve 440 and gas outlet 310, one or more point sensors inserted through sensor access points in body of the gas mixer (e.g. outlet sensor access through-holes 350 in FIG. 3) may sample gas in first flow path 250. Sampling in the gas outlet passage 403 may provide a measurement of gas content after addition of a vaporized agent via the second flow path.

In the example shown in FIG. 4, gas flows through the in-plane s-curves of first mixing passage 406 before flowing through the out-of-plane turn in second mixing passage 430. In other examples, one or more out-of-plane turns may precede one or more s-curves. For example, an additional or alternative flow path may include a first out-of-plane turn followed by one or more in-plane s-curves.

FIG. 5 shows a lateral section view of an example of a gas mixer 500 for an anesthetic vaporizer such as the anesthetic vaporizer 200 illustrated in FIG. 2. Gas mixer 500 may be the same or similar to gas mixer 300 of FIG. 3 and gas mixer 400 of FIG. 4. Therefore, components of gas mixer 500 of FIG. 5 that are identical to components of gas mixer 300 of FIG. 3 and gas mixer 400 of FIG. 4 are numbered the same and will not be reintroduced. For example, gas mixer 500 includes inlet sensor assembly mount 356, inlet sensor access through-holes 346, outlet sensor assembly mount 348 and outlet sensor access through-holes 350. Further, some components of gas mixer 300 and gas mixer 400 may not be shown, although it may be understood that they may also be included in gas mixer 500.

The lateral section view is shown looking into the y axis to illustrate an example of valve seat 502 formed into gas mixer 500. The view further illustrates an example of interior passages of gas mixer 500 including access points for sensors within gas inlet passage 401 and gas outlet passage 403 of the first flow path (250 in FIGS. 2-4) and sections through first mixing passage 406 and second mixing passage 430. Wall 504 illustrates a thickness of portion of gas mixer 500 wherein the first flow path 250 is formed.

A section view of gas inlet passage 401 of the first flow path is shown under inlet sensor assembly mount 356. The section view shows the shape 524 of gas inlet passage 401. The shape may have an upper surface 518 parallel in the xy plane with a lower surface 520 and rounded sides 522, 523 parallel in the yz plane. The shape 526 of first mixing passage 406 may be cylindrical. The shape 528 of gas outlet passage 403 is similar to shape 524. In one example, one or more sensors may be inserted through the plurality of inlet sensor access through-holes 346. Such sensors may sample the gas flows within the first flow path. For example, one or more sensors sampling within the first flow path may take gas measurements before agent is added via junction 407 (in FIG. 4). A section view of gas outlet passage 403 of the first flow path is shown under outlet sensor assembly mount 348. In one example, one or more sensors may be inserted through the plurality of outlet sensor access through-holes 350 for sampling the combined flow within the first flow path after agent is mixed with medical gas or air.

Valve seat 502 may be a substantially cylindrical void having an opening 506. The interior of valve seat 502 comprises a first inner cylindrical void 510 that is concentric with and wider in diameter than a second inner cylindrical void 512. The second inner cylindrical void 512 meets with an annular exterior face 508 of the second flow path 252. The annular exterior face 508 of the second flow path 252 is parallel with an annular exterior face 516 of the second inner cylindrical void 512. An interior surface 514 of second flow path is shown.

Valve seat 502 may be a threaded valve seat for a valve (e.g., valve 244 in FIG. 2). As one example, valve seat 502 may be a seat for a proportional valve. In a vaporizer system, such a valve may control a flow of vaporized agent delivered from a vaporizing chamber (e.g., vaporizing chamber 202 of vaporizer 200 in FIG. 2). The vaporized agent may flow into the gas mixer via second flow path 252 combining with bypass gas in first mixing passage 406 and second mixing passage 430 at and downstream from junction 407 (in FIG. 4). The valve may be adjusted to target a threshold concentration of agent, the concentration determined by comparing sensor signals sampled in the passage before and after junction 407.

FIG. 6 shows a longitudinal section view of an example of a gas mixer 600 for an anesthetic vaporizer such as the anesthetic vaporizer 200 illustrated in FIG. 2. Gas mixer 600 may be the same or similar to gas mixer 300 of FIG. 3, gas mixer 400 of FIG. 4, and gas mixer 500 of FIG. 5. Therefore, components of gas mixer 600 of FIG. 6 that are identical to components of gas mixer 300 of FIG. 3 gas mixer 400 of FIG. 4, and gas mixer 500 of FIG. 5 are numbered the same and will not be reintroduced.

The longitudinal view is a section through second mixing passage 430 and looking into the x axis. Second mixing passage 430 is an out-of-plane turn incorporated into the yz plane. After length 606, the out-of-plane turn begins at curve 428 where the second mixing passage makes a 90 degree turn downwards from the xy plane into the yz plane. Second mixing passage 430 forms a U-shaped bend incorporated in the yz plane and xy plane between curve 428 and curve 432. After vertical length 608, the U-shaped bend may be formed by a substantially 90 degree angled curve 602 followed by length 607 in the xy plane. After length 607, the passage may make a substantially 90 degree angled curve 604 upwards into the yz plane of vertical length 608, mirroring curve 602. At curve 432, the passage makes 90 degree turn back into the xy plane into length 614. The second mixing passage 430 has a first diameter 610. The radius of curvature 612 at curve 432 is similar to first diameter 610.

The view further illustrates an example of second flow path 252. Coupled in a vaporizer system with a vaporizing chamber (e.g., vaporizing chamber 202 in vaporizer 200 of FIG. 2), vaporized agent may flow into second flow path 252 entering the lower passage 616 of the second flow path before flowing through the upper passage 618.

FIG. 7 schematically shows an example of a first mixing passage 700 for in-plane gas mixing. First mixing passage 700 may be the same or similar to first mixing passage 406 of gas mixer 242, 300, 400, 500, and 600 described with respect to the examples shown in FIG. 2, 3, 4, 5, and 6. Components of first mixing passage 700 of FIG. 7 that are identical to components of gas mixer 242, 300, 400, 500, and 600 are numbered the same and will not be reintroduced.

First mixing passage 700 includes a first s-curve 436 and a second s-curve 438. First s-curve 436 includes a first outer radius 706, a second outer radius 708, a first inner radius 710, and a second inner radius 712. Second s-curve 438 includes a third outer radius 714, a fourth outer radius 716, a third inner radius 718, and a fourth inner radius 720.

A first gas stream 702 for a heavier gas (e.g., vaporized agent) indicated with a dashed line and a second gas stream 704 for a mixing gas (e.g., vaporized agent and medical gas) indicated with a solid line are shown combining in first mixing passage 700. Due to the density of the heavier agent, the first gas stream 702 driven by centrifugal forces follows the outer radius of the first mixing passage 700. The in-plane turn at corner or curve 412 causes the first gas stream 702 of heavier agent to cross through the second gas stream 704 to follow second outer radius 708. The first gas stream 702 and the second gas stream 704 cross a second time at curve 416 as heavier gas is drawn from the second outer radius 708 to the third outer radius 714. The first gas stream 702 and the second gas stream 704 cross again at curve 422 as heavier gas is drawn from the third outer radius 714 to fourth outer radius 716. In one example, the effect of s-curved pathway in the first mixing passage is homogenous mixture of a heavier gas with a lighter gas.

FIG. 8 schematically shows an example of a flow path 800 in cross-section 802 illustrating gas flow rotation around a circular bend 804. Circular bend 804 may be the same or similar to a curve in an in-plane s-curves described with respect to the example of gas mixer 242, 300, 400, 500, 600 and 700 in FIGS. 2-7. For example, circular bend 804 may be the same or similar to curve 412 in FIG. 3. A bisecting line 806.

As illustrated in FIG. 8, cross-section 802 is taken across circular bend 804 from A to A. The use of circular cross-section for the s-curves (or serpentine) of first mixing passage results in rotational flow around the corner. Around the corner, gas stream 812 is mixed by rotational flow in the outer radius 810 and the inner radius 808, the flow rotation being caused by the difference between the inner and outer radius pressure differences. To the left of bisecting line 806, gas stream 812 rotates counter clockwise. To the right of bisecting line 806, gas stream 812 rotates clockwise.

FIG. 9 schematically shows an example of second mixing passage 900 for out-of-plane gas mixing. Second mixing passage 900 may be the same or similar to second mixing passage 430 of gas mixer 242, 300, 400, 500, and 600 described with respect to the examples shown in FIG. 2, 3, 4, 5, and 6. Components of second mixing passage 900 of FIG. 9 that are identical to components of gas mixer 242, 300, 400, 500, and 600 are numbered the same and will not be reintroduced.

In some examples, such as following a corkscrew or serpentine passage, a gas stream may be mixed in a laminar flow regime such that well mixed layers form in a gradient. Gas stream 902 is illustrated entering the second mixing passage. Gas stream is mixed in a laminar flow regime in a gradient of percent agent before curve 428. Between curve 428 and curve 432, second mixing passage 900 makes an out-of-plane turn incorporated in the yz plane. The out-of-plane turn forms U-shaped bend 908. As gas stream 902 makes the turn through U-shaped bend 908, the gradient layers are broken up, shown as crossing dashed lines. After curve 432, gas stream 902 is homogenously mixed.

FIG. 10 shows a flow chart of an example method 1000 for detecting a proportion of agent to medical gas in a gas mixture of an anesthetic vaporizer of an anesthesia machine. The anesthetic vaporizer may be the anesthetic vaporizer 200 of FIG. 2, for example. The method 1000 and the rest of the methods included herein may be executed by a controller, such as the controller 225 of FIG. 2, according to instructions stored in a memory of the controller (e.g., the memory 228 of FIG. 2) and in conjunction with one or more inputs, such as inputs received from one or more sensors (e.g., sensors of the first sensor assembly 246 and the second sensor assembly 248 of FIG. 2) or as inputs received from an operator via a human-machine interface (e.g., HMI 226). Further, the controller may output information to an operator of the anesthesia machine via a human-machine interface.

At 1002, the method 1000 includes receiving sensor signals from the anesthetic vaporizer. As described above with respect to FIG. 2, point sensors for detecting thermal conductivity of a gas mixture may be integrated into the first and second sensor assemblies. At least a first point sensor integrated into the first sensor assembly may sense bypass gas in the inlet of the gas mixer. At least a second point sensor integrated into the second sensor assembly may sense mixed gas (e.g., medical gas and agent) in the outlet of the gas mixer. In an additional or alternative example, the first and second sensor assemblies may include one or more optical sensors that transmits light of a suitable wavelength (e.g., infrared) through the mixed gas and determines a concentration of the anesthetic agent based on an absorption of the light by the mixed gas. In another example, the first and second sensor assemblies may include one or more carbon dioxide or oxygen sensors that measure the concentration of the anesthetic agent based on a displacement of the carbon dioxide or oxygen relative to a supplied concentration of carbon dioxide or oxygen in the fresh gas flow.

At 1004, the method 1000 includes calculating an average thermal conductivity signal from gas inlet conductivity sensors. For example, the average thermal conductivity signal may be sampled over a duration and averaged. Additionally or alternatively, the average thermal conductivity signal may be sampled at more than one sensor.

At 1006, the method 1000 includes calculating an average thermal conductivity signal from gas outlet conductivity sensors. As above, the average thermal conductivity signal may be sampled over a duration and averaged and may additionally or alternatively include outlet gas mixture sampling at more than one sensor.

At 1008, the method 1000 includes determining a concentration of agent in the gas mixture based on a difference in average thermal conductivity of the gas flow measured at the first sensor assembly compared to the average thermal conductivity of the gas flow at second sensor assembly. In one example, by knowing the thermal conductivity of the gases (e.g., an intrinsic property), the proportion of gases (e.g., medical gas to vaporized agent) in the mixture may be determined. The signal difference in thermal conductivity between the inlet gas mixture and the outlet gas mixture is proportional to the amount of agent in the mixture.

At 1010, the method 1000 includes determining if the proportion of agent to mixed gas is within a threshold range. For example, the controller may compare the signal difference to a threshold signal difference. As one example, the controller may compare the measured thermal conductivity difference to an expected conductivity for gas with the agent at the desired concentration. The expected conductivity may be determined by reference to a look-up table.

In response to the agent to mixed gas proportion being outside of the threshold range, or in other words greater than a threshold difference, the method 1000 proceeds to 1012 and includes generating an indication. The indication may be output by the HMI, for example, as an audible and/or visual alert (or alarm). For example, the audible alert may include an alarm sound that is output via speakers of the HMI. Additionally or alternatively, the audible alert may include a spoken message including the concentration of agent. Similarly, the visual alert may include the concentration of agent and other instructions.

At 1014, the method 1000 includes automatic adjustment of the valve to vaporized agent. For example, the controller may adjust a throttle of the vaporizer tank valve to allow more or less vaporized agent into the second mixing passage using feedback control from the point sensors integrated into the gas mixer. In other examples of the method 1000, at 1014 automatic adjustment may be omitted and an operator may give instructions to the controller to adjust the amount of anesthetic agent delivered to a patient. For example, the controller may receive the desired concentration of the anesthetic agent via input device and may determine an amount of opening of one or more valves corresponding to the desired concentration of the anesthetic agent based on the lookup table, with the input being the concentration of the anesthetic agent and the output being the valve position of the one or more valves. The controller may transmit an electrical signal to an actuator of the one or more valves in order to adjust each of the one or more valves to the corresponding output valve position.

The method 1000 may then return. For example, the method 1000 may be repeated at throughout vaporizer operation.

Thus, the systems and methods described herein provide for homogenous mixture of gases over a very short mixing length including over a large dynamic flow range and with reduced pressure drop. As one advantage, by achieving homogenous mixture over short mixing the use of point concentration sensors may be enabled, supporting more compact vaporizer design. Moreover, the systems and methods enable quick and verifiable changes in agent concentration of delivered gas and feedback control of anesthetic gas delivery.

A technical effect of using in-plane and out-of-plane mixing to achieve homogenous mixture over a very short mixing length is more responsive control of gas mixing in an anesthesia system and reduced likelihood of patient discomfort.

The disclosure also provides support for a system for a vaporizer gas mixer comprising: a one-piece body, a gas inlet passage and a gas outlet passage, a first flow path fluidically coupled to the gas inlet passage and the gas outlet passage, the first flow path having a plurality of curves, and a second flow path fluidically coupled to the first flow path and a vaporizing chamber, wherein the first flow path and the second flow path merge after the gas inlet passage and before the plurality of curves. In a first example of the system, the plurality of curves further comprises a first mixing passage and a second mixing passage, wherein the first mixing passage comprises an s-curve in-plane with respect to the gas inlet passage and the gas outlet passage. In a second example of the system, optionally including the first example, the first mixing passage comprises at least two s-curves in-plane with respect to the gas inlet passage and the gas outlet passage. In a third example of the system, optionally including one or both of the first and second examples, the second mixing passage comprises a curve out-of-plane with respect to the gas inlet passage and the gas outlet passage. In a fourth example of the system, optionally including one or more or each of the first through third examples, gas flows from the vaporizing chamber into the second flow path is actuated by a valve. In a fifth example of the system, optionally including one or more or each of the first through fourth examples, one or more sensors are positioned in the gas inlet passage to sense gas flows in first flow path before being combined with gas flows from the second flow path. In a sixth example of the system, optionally including one or more or each of the first through fifth examples, one or more point sensors are positioned in the gas outlet passage to sense gas flows in the first flow path after being combined with gas flows in the second flow path.

The disclosure also provides support for a system, comprising: an anesthetic source of anesthetic gas, a vaporizer gas mixer having a one-piece plastic body coupled with the anesthetic source, the one-piece plastic body having a gas inlet passage and a gas outlet passage in a first plane with a flow path therebetween, the flow path having a first region curving only in the first plane and a second region curving only out of the first plane. In a first example of the system, the one-piece plastic body further coupled with a source of medical gas. In a second example of the system, optionally including the first example, anesthetic gas enters the flow path through an opening in an internal wall of the flow path, the opening positioned in the flow path before the first region and the second region. In a third example of the system, optionally including one or both of the first and second examples, gas flows from the source of medical gas enter the vaporizer gas mixer through the gas inlet passage and combines flow with the anesthetic source of anesthetic gas at and downstream from the opening, the combined flow exiting the vaporizer gas mixer through the gas outlet passage. In a fourth example of the system, optionally including one or more or each of the first through third examples, curves of the first region and the second region are in a central region of the one-piece plastic body. In a fifth example of the system, optionally including one or more or each of the first through fourth examples, the vaporizer gas mixer further having a plurality of through-holes for at least a first sensor and at least a second sensor. In a sixth example of the system, optionally including one or more or each of the first through fifth examples, the first sensor is positioned in the gas inlet passage before medical gas combines with anesthetic gas and the second sensor is positioned in the gas outlet passage after the first region and the second region of the flow path. In a seventh example of the system, optionally including one or more or each of the first through sixth examples, the second region comprises a protrusion out-of-plane of the one-piece plastic body. In an eighth example of the system, optionally including one or more or each of the first through seventh examples, the gas flows between the anesthetic source of anesthetic gas and the one-piece plastic body are controlled by an actuator. In a ninth example of the system, optionally including one or more or each of the first through eighth examples, the flow path comprises a plurality of s-curves, the first region having at least two s-curves in-plane and the second region having at least one s-curve out-of-plane. In a tenth example of the system, optionally including one or more or each of the first through ninth examples, the flow path from the gas inlet passage to the gas outlet passage comprises a smooth internal wall. In an eleventh example of the system, optionally including one or more or each of the first through tenth examples, the flow path narrows after the opening in the internal wall of the flow path and before the plurality of s-curves. In a twelfth example of the system, optionally including one or more or each of the first through eleventh examples, the system further comprises: a controller storing executable instructions in non-transitory memory that, when executed cause the controller to: receive signals from the first sensor and the second sensor, compare a signal difference of the first sensor and the second sensor to a threshold signal difference, and, responsive to identifying signal difference greater than a threshold difference, adjust the actuator controlling the flow of anesthetic gas between the anesthetic source and the one-piece plastic body.

FIGS. 3-6 are shown approximately to scale. As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plainlanguage equivalents of the respective terms "comprising" and "wherein." Moreover, the terms "first," "second," and "third," etc. are used merely as labels, and are not intended to impose numerical requirements or a particular positional order on their objects.

As used herein, the term "substantially" is construed to mean plus or minus five percent of the range unless otherwise specified.

This written description uses examples to disclose the invention, including the best mode, and also to enable a person of ordinary skill in the relevant art to practice the invention, including making and using any devices or systems and performing any incorporated methods. The patentable scope of the invention is defined by the claims.
Other examples are intended to be within the scope of the claims if they have structural elements that do not differ from the literal language of the claims, or if they include equivalent structural elements with insubstantial differences from the literal languages of the claims.

## Claims

1. A vaporizer gas mixer (300) having a one-piece body arranged to be coupled with an anesthetic source of anesthetic gas (210), the one-piece body having a gas inlet passage (401) and a gas outlet passage (403) in a first plane with a flow path (250) therebetween, the flow path (250) having a first region (406) curving only in the first plane and a second region (430) curving only out of the first plane, wherein the gas inlet passage (401) is arranged to be coupled with a source of medical gas (146, 148) and an internal wall of the flow path has an opening (407) arranged to allow anesthetic gas from the anesthetic source of anesthetic gas (210), to enter the flow path (250), the opening being positioned in the flow path before the first region (468) and the second region (470).

2. The vaporizer gas mixer (300) of claim 1, arranged so that gas flowing from the source of medical gas (146, 148) and entering the vaporizer gas mixer (300) through the gas inlet passage (401) combines flow with anesthetic gas from the anesthetic source of anesthetic gas (210) at and downstream from the opening (407), the combined flow exiting the vaporizer gas mixer (300) through the gas outlet passage (403).

3. The vaporizer gas mixer (300) of claim 2, wherein curves of the first region (468) and the second region (470) are in a central region (466) of the one-piece body.

4. The vaporizer gas mixer (300) of claim 3, the vaporizer gas mixer (300) further having a plurality of through-holes (346, 350) for at least a first sensor and at least a second sensor.

5. The vaporizer gas mixer (300) of claim 4, wherein the first sensor is positioned in the gas inlet passage (401) before the opening (407) and the second sensor is positioned in the gas outlet passage (403) after the first region (468) and the second region (470) of the flow path (250).

6. The vaporizer gas mixer (300) of claim 5, wherein the second region (470) comprises a protrusion (430) out-of-plane of the one-piece body.

7. The vaporizer gas mixer (300) of any preceding claim wherein the on-piece body os plastic.

8. A system comprising the vaporizer gas mixer (300) of any preceding claim and the anesthetic source of anesthetic gas (210).

## Patentansprüche

1. Verdampfergasmischer (300) mit einem einteiligen Körper, der zur Verbindung mit einer Anästhesiequelle für Narkosegas (210) ausgelegt ist, wobei der einteilige Körper einen Gaseinlasskanal (401) und einen Gasauslasskanal (403) in einer ersten Ebene mit einem Strömungsweg (250) dazwischen aufweist, wobei der Strömungsweg (250) einen ersten Bereich (406), der sich nur in der ersten Ebene krümmt, und einen zweiten Bereich (430), der sich nur aus der ersten Ebene heraus krümmt, aufweist, wobei der Gaseinlasskanal (401) zur Verbindung mit einer Quelle für medizinisches Gas (146, 148) angeordnet ist, und eine Innenwand des Strömungswegs eine Öffnung (407) aufweist, die dazu angeordnet ist, Narkosegas aus der Anästhesiequelle für Narkosegas (210) in den Strömungsweg (250) eintreten zu lassen, wobei die Öffnung im Strömungsweg vor dem ersten Bereich (468) und dem zweiten Bereich (470) angeordnet ist.

2. Verdampfergasmischer (300) nach Anspruch 1, der so angeordnet ist, dass Gas, das aus der Quelle für medizinisches Gas (146, 148) strömt und durch den Gaseinlasskanal (401) in den Verdampfergasmischer (300) eintritt, den Strom mit Narkosegas aus der Anästhesiequelle für Narkosegas (210) an und stromabwärts von der Öffnung (407) kombiniert, wobei der kombinierte Strom den Verdampfergasmischer (300) durch den Gasauslasskanal (403) verlässt.

3. Verdampfergasmischer (300) nach Anspruch 2, wobei Kurven des ersten Bereichs (468) und des zweiten Bereichs (470) in einem zentralen Bereich (466) des einteiligen Körpers liegen.

4. Verdampfergasmischer (300) nach Anspruch 3, wobei der Verdampfergasmischer (300) ferner eine Vielzahl von Durchgangslöchern (346, 350) für mindestens einen ersten Sensor und mindestens einen zweiten Sensor aufweist.

5. Verdampfergasmischer (300) nach Anspruch 4, wobei der erste Sensor in dem Gaseinlasskanal (401) vor der Öffnung (407) angeordnet ist und der zweite Sensor in dem Gasauslasskanal (403) nach dem ersten Bereich (468) und dem zweiten Bereich (470) des Strömungswegs (250) angeordnet ist.

6. Verdampfergasmischer (300) nach Anspruch 5, wobei der zweite Bereich (470) einen Vorsprung (430) außerhalb der Ebene des einteiligen Körpers umfasst.

7. Verdampfergasmischer (300) nach einem der vorhergehenden Ansprüche, wobei der einteilige Körper aus Kunststoff besteht.

8. System, umfassend den Verdampfergasmischer (300) nach einem der vorhergehenden Ansprüche und die Anästhesiequelle für Narkosegas (210).

## Revendications

1. Mélangeur de gaz à vaporisateur (300) comportant un corps monobloc agencé pour être couplé à une source anesthésique de gaz anesthésique (210), le corps monobloc comportant un passage d'entrée de gaz (401) et un passage de sortie de gaz (403) dans un premier plan avec un trajet d'écoulement (250) entre eux, le trajet d'écoulement (250) comportant une première région (406) qui se courbe uniquement dans le premier plan et une deuxième région (430) qui se courbe uniquement hors du premier plan, le passage d'entrée de gaz (401) étant agencé pour être couplé à une source de gaz médical (146, 148) et une paroi interne du trajet d'écoulement comportant une ouverture (407) agencée pour permettre au gaz anesthésique provenant de la source anesthésique de gaz anesthésique (210) d'entrer dans le trajet d'écoulement (250), l'ouverture étant positionnée dans le trajet d'écoulement avant la première région (468) et la deuxième région (470).

2. Mélangeur de gaz à vaporisateur (300) selon la revendication 1, agencé de telle sorte que le gaz s'écoulant de la source de gaz médical (146, 148) et entrant dans le mélangeur de gaz à vaporisateur (300) par le passage d'entrée de gaz (401) se combine avec le gaz anesthésique provenant de la source anesthésique de gaz anesthésique (210) au niveau et en aval de l'ouverture (407), le flux combiné sortant du mélangeur de gaz à vaporisateur (300) par le passage de sortie de gaz (403).

3. Mélangeur de gaz à vaporisateur (300) selon la revendication 2, les courbes de la première région (468) et de la deuxième région (470) se trouvant dans une région centrale (466) du corps monobloc.

4. Mélangeur de gaz à vaporisateur (300) selon la revendication 3, le mélangeur de gaz à vaporisateur (300) comportant en outre une pluralité de trous traversants (346, 350) pour au moins un premier capteur et au moins un deuxième capteur.

5. Mélangeur de gaz à vaporisateur (300) selon la revendication 4, le premier capteur étant positionné dans le passage d'entrée de gaz (401) avant l'ouverture (407) et le deuxième capteur étant positionné dans le passage de sortie de gaz (403) après la première région (468) et la deuxième région (470) du trajet d'écoulement (250).

6. Mélangeur de gaz à vaporisateur (300) selon la revendication 5, la deuxième région (470) comprenant une saillie (430) hors du plan du corps monobloc.

7. Mélangeur de gaz à vaporisateur (300) selon l'une quelconque des revendications précédentes, le corps monobloc étant en plastique.

8. Système comprenant le mélangeur de gaz à vaporisateur (300) selon l'une quelconque des revendications précédentes et la source anesthésique de gaz anesthésique (210).
